# EUROPEAN PATENT SPECIFICATION

(11) **EP 2 618 770 B1**
(45) Date of publication and mention of the grant of the patent: **18.12.2019**
(21) Application number: 11827418.2
(22) Date of filing: 21.09.2011
(51) Int. Cl.: A61L 31/04, A61L 15/22, B32B 27/00, A61F 13/36, B32B 5/02

(54) **MEDICAL TOOL DEFLECTION AND RESISTANT MEDICAL SPONGE**
GEGEN ABLENKUNG DURCH MEDIZINISCHE WERKZEUGE RESISTENTER MEDIZINISCHER SCHWAMM
DÉFORMATION D'UN OUTIL MÉDICAL ET ÉPONGE MÉDICALE RÉSISTANTE

(30) Priority: 09.09.2011 US 201113229195; 22.09.2010 US 385346 P
(43) Date of publication of application: 31.07.2013
(73) Proprietor: American Surgical Sponges, LLC, Lynn, Massachusetts 01902 (US); Shao, Weiru, Auburndale, Massachusetts 02466 (US); Piasio, Erik, Salem, Massachusetts 01970 (US)
(72) Inventor: SHAO, Weiru, Auburndale, Massachusetts 02466 (US); PIASIO, Erik, Salem, Massachusetts 019070 (US)
(74) Representative: Prinz & Partner mbB
(86) International application number: PCT/US2011/052494
(87) International publication number: WO 2012/040291

(56) References cited:
- WO-A2-93/24086
- WO-A2-2012/054464
- US-A- 3 911 922
- US-A- 4 068 666
- US-A- 4 068 666
- US-A- 4 961 735
- US-A- 5 112 325
- US-A- 5 460 621
- US-A- 5 556 391
- US-A1- 2005 165 445

## Description

### CROSS REFERENCE TO RELATED APPLICATION

This application claims benefit of U.S. 2011/3229195 entitled, 'Medical Tool Deflection and Resistant Surgical Sponge,' filed September 22, 2010.

### FIELD OF THE DISCLOSURE

The present disclosure is generally related to medical sponges and more particularly is related to a medical tool deflection and resistant medical sponge.

### BACKGROUND OF THE DISCLOSURE

Many surgeries require the use of medical tools. For example, in many keyhole surgeries, such as microsurgery, i.e., ear cranial and spine surgeries, drilling of bone with a medical drill, often known as a burr or dissecting tool, immediately next to critical structures such as neural or vascular structures is frequently required. Conventional techniques include placing strips of surgical sponges on top of the critical structures for protection and hydration. Sometimes, however, the working space proximate to the critical structures is extremely limited and surgical tools contact the sponges. Although this may not be a problem in some operations, in others it can cause significant harm to the patient, as well as cause delays in the surgery.

For example, when a medical drill is needed to remove bone to access underlying structures, the rotating bit may be used proximate to the surgical sponge. If the spinning drill bit accidentally contacts the sponge, the fibers within the surgical sponge may catch on to the features of the drill bit and become entangled with the bit. This results in the surgical sponge balling up around or wrapping around the tip of the rotating dissecting tool or drill bit, which causes significant damage to the adjacent surgical setting. For instance, if this were to happen during a surgery near open brain tissue, the spinning surgical sponge attached to the drill bit may contact the brain tissue and cause a brain injury or contamination of that surrounding area. This may not only cause complications with the operation or surgical procedure, but it may cause additional damage to the patient, which could result in substantial injuries, complications, and even fatalities.

WO93/24086 A2 describes a medical sponge made of compressed textile fibers covered up on at least one side with a thin layer of soft, porous, non absorbent and non adherent material. The thin layer shall ensure that the sponge does not adhere to body tissue.

Thus, a heretofore unaddressed need exists in the industry to address the aforementioned deficiencies and inadequacies.

### SUMMARY OF THE DISCLOSURE

Embodiments of the present disclosure provide a medical sponge. Briefly described, in architecture, one embodiment of the system, among others, can be implemented as follows. The medical sponge comprises a first layer constructed from a biocompatible absorption material, wherein the absorption material includes a woven or non-woven material. A second layer is positioned abutting the first layer, the second layer constructed from a non-toxic, biocompatible deflection material that includes polytetrafluoroethylene (PTFE). A bonding element is connected to the first layer and the second layer, wherein the bonding element adheres the first layer and the second layer together. The bonding element is selected from the group consisting of: an ultrasonic welding joint, a plurality of stitches, or an adhesive-based bonding material, such as glues, or adhesive compounds.

An exemplary method of preventing medical drill damage with a medical sponge during a medical operation can be broadly summarized by the following steps: providing the medical sponge having a first layer constructed from a biocompatible absorption material; bonding a second layer constructed from a deflection material to the first layer; placing the medical sponge proximate to at least one critical structure of a surgical patient; and deflecting a surgical tool with the second layer.

An exemplary method of constructing a medical sponge can be broadly summarized by the following steps: positioning a first layer constructed from a biocompatible, fibrous, absorption material proximate to a second layer, wherein the second layer is constructed from a non-toxic, non-fibrous, biocompatible deflection material; and bonding the first layer to the second layer with a bonding element.

Other features and advantages of the present disclosure will be or become apparent to one with skill in the art upon examination of the following drawings and detailed description. It is intended that all such additional features and advantages be included within this description, be within the scope of the present disclosure, and be protected by the accompanying claims.

### BRIEF DESCRIPTION OF THE DRAWINGS

Many aspects of the disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.
FIG. 1 is a cross-sectional illustration of a medical sponge, in accordance with a first exemplary embodiment of the present disclosure.
FIG. 2 is a cross-sectional illustration of a medical sponge, in accordance with the first exemplary embodiment of the present disclosure.
FIG. 3 is a plan view illustration of a medical sponge, in accordance with the first exemplary embodiment of the present disclosure.
FIG. 4 is a cross-sectional illustration of a medical sponge in use with a medical patient, in accordance with the first exemplary embodiment of the present disclosure.
FIG. 5 is a cross- sectional illustration of a medical sponge, in accordance with a second exemplary embodiment of the present disclosure.
FIG. 6 is a flowchart illustrating an exemplary method of preventing damage with a medical sponge during a medical operation.
FIG. 7 is a flowchart illustrating an exemplary method of constructing a medical sponge.

### DETAILED DESCRIPTION

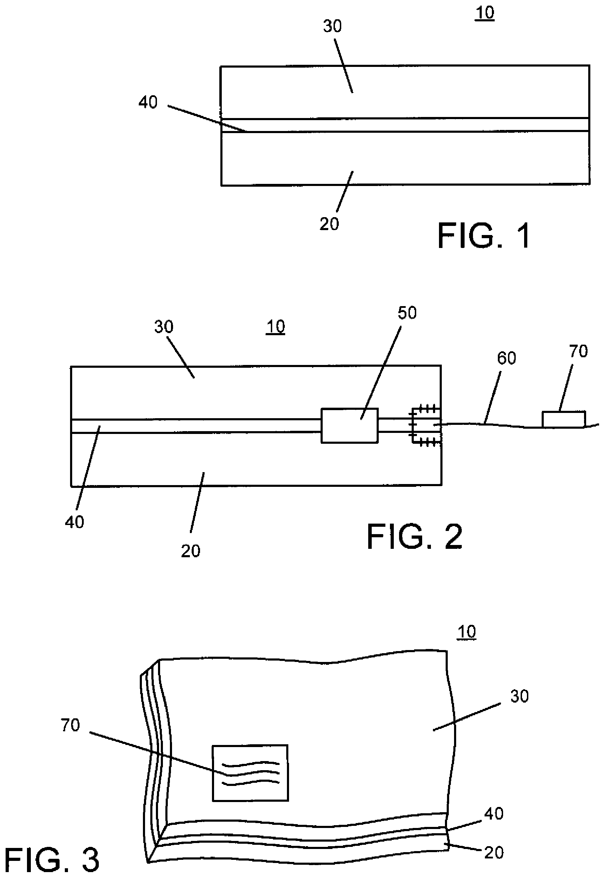

FIG. 1 is a cross-sectional illustration of a medical sponge 10, in accordance with a first exemplary embodiment of the present disclosure. The medical sponge 10, which may be referred to herein simply as 'sponge 10,' includes a first layer 20 constructed from a biocompatible absorption material. A second layer 30 is positioned abutting the first layer 20. The second layer 30 is constructed from a non-toxic, biocompatible deflection material. A bonding element 40 is connected to the first layer 20 and the second layer 30. A bonding element 40 adheres the first layer 20 and the second layer 30 together.

The medical sponge 10 may be used in any medical procedure in any medical field, including surgeries, examinations, and operations on any human being or other living being. Commonly, the medical sponge 10 may be used in surgeries or operations that are proximate to 'critical structures' or 'critical areas' in the patient. A critical structure or area may be any part of a patient's body, where inadvertent contact or disruption may cause damage to the patient. These critical structures may include nerves, brain tissue, blood vessels, or other organs, such as the eyes, ears, or mouth. Disruption of the critical area may cause complications with the surgical procedure, such as additional medical harm to the patient and even the risk of fatalities.

For example, the medical sponge 10 may have a particularly beneficial use in surgeries performed in key-hole fashion, where bone or cartilage must be drilled away to provide surgical exposure or decompression. These surgeries may have a high risk, though, since the drilling location may be very close to the critical structures of the patient. The medical sponge 10 may offer benefits in preventing damage to a patient in any medical procedure that uses a medical or surgical tool, including a drill, often known as a burr or dissecting tool, a scalpel, lancet, knife, medical blade, sickle knife, fine-tip suction, sharp pick and other surgical instruments or object. Although surgeries proximate to critical structures may be common, the medical sponge 10 may also be used with a surgery proximate to any other part of the patient' s body.

As medical standards may require, the medical sponge 10 may be a sterile product that is stored and transported in a sterile packaging. Accordingly, the medical sponge 10 may be constructed in a sterile environment and used in a sterile environment, such as a sterile operating room. The medical sponge 10 may come in any variety of shapes and sizes, as desired by a medical professional or as required depending on the type of medical procedure. It is preferable for the medical sponge 10 to be approximately the same thickness as conventional sponges used within the medical field. This may allow the medical sponge 10 to easily and conveniently be used in locations where operating space is lacking. In addition, the medical sponge 10 may provide benefits in hydrating a location proximate to the surgical procedure, for removing excess bodily fluids, or for preventing desiccation of the critical structure of the patient. The medical sponge 10 may be moistened prior to use if needed, which includes moistened during a packaging process or moistened after the medical sponge 10 is removed from a packing container.

The first layer 20 of the medical sponge 10 is constructed from a biocompatible absorption material. The biocompatible absorption material may include any material that is conventionally used in medical sponges, such as absorbent or hydrating fibrous materials. The first layer 20 is biocompatible, so it can be used on a patient with an opening in their body without causing harm to the patient, or creating a negative reaction within the patient. The biocompatible absorption material includes a cotton woven or non-woven material, with any number of plys and fabrics. Other materials may include any combination of polyester, cellulose, foam, or any other absorbent material, all of which are considered within the scope of the present disclosure.

The second layer 30 is positioned to abut the first layer 20 to form the general structure of the medical sponge 10. The abutment between the second layer 30 and the first layer 20 may be retained by a bonding element 40. The bonding element 40 may include any number of bonding devices or methods, including those conventionally used within the field. For example, the bonding element 40 may include an ultrasonic welding joint between the first layer 20 and the second layer 30 or a number of stitches between the first layer 20 and the second layer 30. Other bonding elements 40 may include adhesive-based bonding materials, such as glues, adhesive compounds, and the like. It is noted that the bonding element 40 may be present between all of the abutting surfaces of the first layer 20 and the second layer 30, or only a portion thereof. For example, the bonding element 40 may be an adhesive that is positioned fully between all contacting surfaces of the first layer 20 and the second layer 30, or the bonding element 40 may be stitching that only retains the edges of the first layer 20 and the second layer 30 together. Naturally, other configurations not explicitly stated herein may be used as well. The second layer 30 is constructed from a non-toxic, biocompatible deflection material. Similar to the properties of the first layer 20, the deflection material of the second layer 30 is biocompatible and non-toxic so it does not risk contaminating or otherwise harming a patient that the medical sponge 10 is used on. Additionally, the deflection material must be capable of deflecting a medical tool. The deflection material includes polytetrafluoroethylene (PTFE), commonly known under the trade name TEFLON®. This PTFE material may be substantially dense, yet have a low friction exterior, such that when a drill bit or scalpel inadvertently touches the second layer 30 of the medical sponge 10, the drill bit or scalpel is unable to penetrate through the second layer 30. Other materials beyond PTFE may provide the same characteristics as PTFE and be used with the medical sponge 10. It is preferable for the deflection material of the second layer 30 to be non-fibrous, such that there is a lack of fibers, textures, gaps, or other structures that a rotating drill bit or moving scalpel to catch on.

One of the benefits of the medical sponge 10 is that the use of the second layer 30 having the deflection material may prevent entanglement of the absorption materials of the first layer 20 with a medical tool. For example, a surgeon may use a rotating drill bit proximate to the sponge and may inadvertently or accidentally contact the medical sponge 10 with the rotating drill bit. When this happens with a conventional sponge, the fibrous materials that contact the rotating drill bit would be removed from over the critical structure and immediately wrap around the spinning drill bit. The spinning material would subsequently contact the critical area that it used to be covering and cause significant damage to it. Thus, the conventional sponge that was intended to protect the critical area may easily cause more harm to it.

Furthermore, the surgeon would then have to remove the tangled material and reapply more absorption material to the critical area.

The medical sponge 10 of the present disclosure prevents any entanglement of the absorption materials of the first layer 20 by deflecting the drill bit or other tool. In the example above, when the surgeon inadvertently contacts the spinning drill bit with the second layer 30 of the medical sponge 10, the deflection material prevents any entanglement with the medical sponge 10 and the drill bit. Thus, contact of the drill bit and the second layer 30 may slightly depress or bias the medical sponge 10, but it does not remove the medical sponge 10 or move it in any substantial way. Similarly, when a scalpel or other sharp object is used near the medical sponge 10, the second layer 30 may prevent it from catching on the absorption materials of the first layer 20. In addition, when multiple conventional sponges are used and become tangled, medical staff looses the ability to count the sponges. This increases the risk of a retained foreign object within the patient's body, and may cause subsequent complications. The ability to prevent entanglement with the medical sponge 10 of the present disclosure may lessen this risk.

It is noted that even though the second layer 30 is not intended to contact the patient, when a rotating drill bit contacts the second layer 30, microscopic particles of the deflection material may be removed from the second layer 30. This does not move or relocate the medical sponge 10, and may often go unnoticed by the surgeon.

However, the deflection material should be biocompatible because these microscopic particles may be spread around the proximate area, and may end up entering the patient's body.

In use, the surgeon, the surgeon's assistant, or other medical professional may place the medical sponge 10 proximate or directly next to a critical area to collect blood or other bodily fluid that may be released, or to keep the area hydrated by preventing unwanted drying out of the area due to it's exposure. As can be seen, the medical sponge 10 may keep the surgical area substantially free from blood and fluid, thereby allowing the surgeon more visibility and access to the surgical area, or the medical sponge 10 may keep a critical area hydrated. The surgeon may then operate on the patient using any tools needed. If a tool contacts the medical sponge 10, the second layer 30 will deflect the tool, allowing the surgeon to carry on with the surgery. Of course, use of the medical sponge 10 may be consistent with medical techniques, such as replacing the medical sponge 10 when it has reached its absorption capacity or become dehydrated, respectively. Once the surgical procedure is completed, the medical sponge 10 may be removed and disposed of accordingly.

It is further noted that the medical sponge 10 is described with the first layer 20 bonded to the second layer 30 with the bonding element 40, but that additional layers or material may be included between the first layer 20 and the second layer 30 without departing from the scope of the present disclosure. For example, the first layer 20 may include multiple absorbent materials that are layered together and bonded to the second layer 30. Likewise, other layers of materials may be placed between the first layer 20 and the second layer 30. If this occurs, the bonding element 40 may bond the first layer 20 to the second layer 30 through the additional layers located therebetween.

FIG. 2 is a cross-sectional illustration of a medical sponge 10, in accordance with the first exemplary embodiment of the present disclosure. The medical sponge 10 of FIG. 2 includes the features discussed with respect to FIG. 1 and some additional features that may be included with the medical sponge 10. For example, the medical sponge 10 includes a radiopaque marker 50 which may be embedded or otherwise affixed to the medical sponge 10. The radiopaque marker 50 may include any material that substantially prevents radiation penetrate, such as radiation from X-rays. As one having skill in the art would recognize, this allows the surgeon to account for all medical sponges 10 used within a particular medical operation, thereby preventing any accidental situations where a medical sponge 10 is left within a patient's body after the surgical procedure.

Also shown in FIG. 2 is a positioning element 60 attached to the medical sponge 10. The positioning element 60 may include any type of positioning structure, such as flexible string or a substantially rigid tab (not shown) constructed from a medically safe material. The positioning element 60 may allow the surgeon or medical assistant to position or reposition the medical sponge 10 as needed. It may also allow for removal of the medical sponge 10. The positioning element 60 may include any design, may be any size, and may be affixed to the medical sponge 10 in any configuration. For example, the positioning element 60 may be affixed to an interior portion of the medical sponge 10, such as between the first layer 20 and the second layer 30, as is shown in FIG. 2, or it may be affixed to the exterior of the medical sponge 10.

An identification element 70 may also be included with the medical sponge 10 to allow for identification of a characteristic or property of the sponge. The identification element 70 may be affixed to the positioning element 60, as is shown in FIG. 2, or it may be affixed elsewhere on the medical sponge 10, as is discussed with respect to FIG. 3. The identification element 70 may include any serial number or character(s), any color, any design, or other identifying mark. For example, the identification element 70 may be a brightly colored marker that easily shows the position of the medical sponge 10. The identification element 70 may also indicate the number of medical sponges 10 used in a procedure, i.e., by indicating that the medical sponge 10 is 'sponge 7 of 10,' or the type of medical sponge 10, such as the absorption or deflection properties of the medical sponge 10. Of course, other features and components that are commonly found in use with conventional sponges may also be used with the medical sponge 10, all of which are considered within the present disclosure.

FIG. 3 is a plan view illustration of a medical sponge 10, in accordance with the first exemplary embodiment of the present disclosure. The medical sponge 10 is shown with the identification element 70 positioned on the exterior of the medical sponge 10. Accordingly, the identification element 70 may be positioned on any surface of the medical sponge 10, including the surface of the first layer 20, the second layer 30 (as shown), or any cross-sectional surface. The identification element 70 may also be retained with the bonding element 40, such as an adhesive that retains the identification element 70 to the side of the medical sponge 10, or stitching that retains the identification element 70 to a surface of the medical sponge 10.

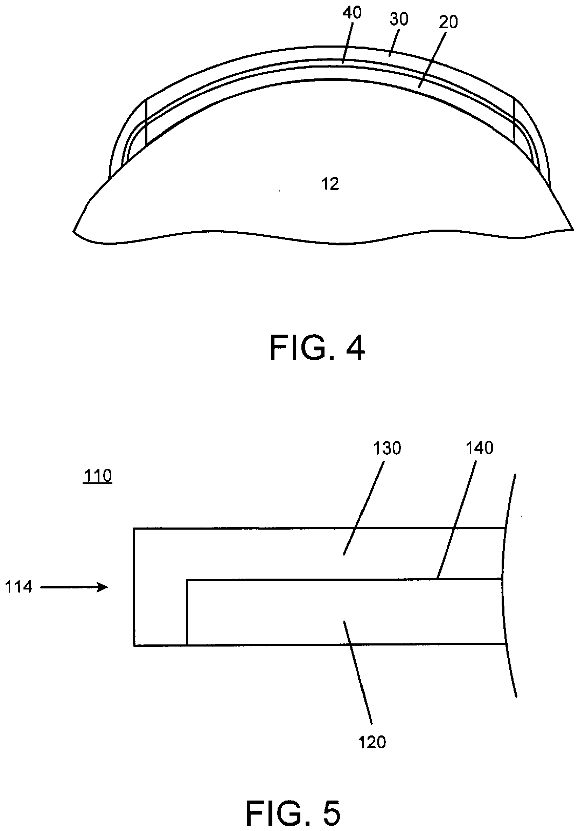

FIG. 4 is a cross-sectional illustration of a medical sponge 10 in use with a medical patient 12, in accordance with the first exemplary embodiment of the present disclosure. As mentioned with respect to FIG. 1, the overall size of the medical sponge 10 is an important consideration during medical procedures, since the surgical area and proximate space is frequently limited. Accordingly, it is beneficial for the medical sponge 10 to have a relatively small thickness, such as the thickness that conventional medical operation sponges have. Furthermore, the medical sponge 10 may have an overall structure that allows it to be used with a variety of medical procedures and/or on a variety of surgical sites. In particular, the medical sponge 10 should be rigid enough to successfully deflect the drill bit, but should not be overly rigid where the medical sponge 10 is incapable of draping over the surgical site or conforming to the critical area.

In FIG. 4, the medical sponge 10 is shown in an in-use position with the medical patient 12, such that the medical sponge 10 is positioned to abut critical areas of the medical patient 12, including the patient's eyes or mouth. As is shown in FIG. 4, the first layer 20, second layer 30, and boning layer 40 of medical sponge 10 may be constructed to be flexible enough to conform to the shape or features of the medical patient 12. The ability to drape the medical sponge 10 over any structure of the medical patient 12, having any surface features, may allow the medical sponge 10 to be conveniently used on any part of the medical patient 12 without significant manipulation or adjustment.

FIG. 5 is a cross-sectional illustration of a medical sponge 110, in accordance with a second exemplary embodiment of the present disclosure. The medical sponge 110 is substantially similar to the medical sponge 10 of the first exemplary embodiment, and may include any of the features or components discussed with respect to the first exemplary embodiment. Accordingly, the medical sponge 110 includes a first layer 120 constructed from a biocompatible absorption material. A second layer 130 is positioned abutting the first layer 120. The second layer 130 is constructed from a non-toxic, biocompatible deflection material. A bonding element 140 is connected to the first layer 120 and the second layer 130. A bonding element 140 adheres the first layer 120 and the second layer 130 together.

As is shown in FIG. 5, the second layer 130 may be configured to cover an edge 114 of the medical sponge 110. For example, the first layer 120 may be slightly smaller in size than the second layer 130. The edges of the second layer 130 may then be folded or configured to cover the edges of the first layer 120. This may create a medical sponge 110 that includes deflection material on the sides of the medical sponge 110, in addition to one of the surfaces. When the medical sponge 110 is in use, the second layer 130 having deflection material on the edge 114 of the medical sponge 110 may prevent a medical tool that contacts the edge 1 14 of the medical sponge 110 from becoming entangled with the first layer 120. Other variations of this design are considered within the scope of the present disclosure.

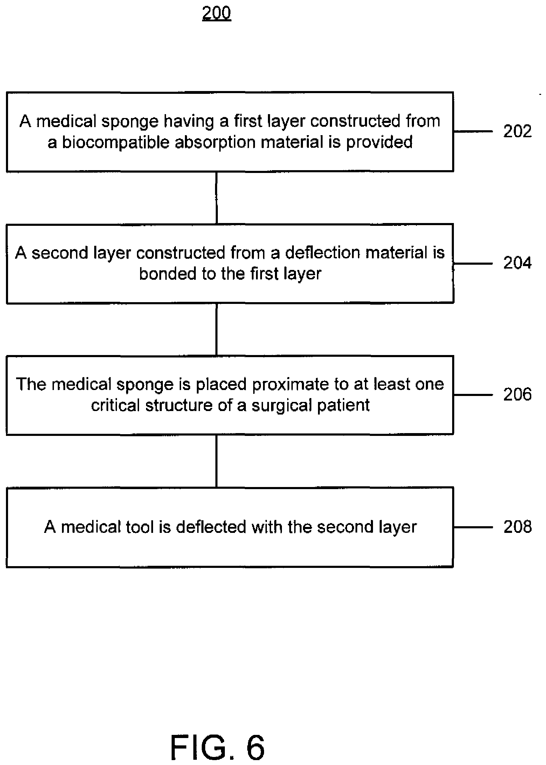

FIG. 6 is a flowchart 200 illustrating a exemplary method of preventing damage with a medical sponge during a medical operation. It should be noted that any process descriptions or blocks in flow charts should be understood as representing modules, segments, portions of code, or steps that include one or more instructions for implementing specific logical functions in the process.

As is shown by block 202, a medical sponge having a first layer 20 constructed from a biocompatible absorption material is provided. A second layer 30 constructed from a deflection material is bonded to the first layer 30 (Block 204). The medical sponge 10 is placed proximate to at least one critical structure of a surgical patient (Block 206). A medical tool is deflected with the second layer 30 (Block 208).

Deflecting the medical tool with the second layer may include deflecting a drill bit and/or a scalpel. This may include contacting a surface of the second layer with the medical tool and preventing the medical tool from traversing through the second layer. Throughout or during any deflection of the medical tool, the medical sponge may be retained proximate to the at least one critical structure of the surgical patient. Depending on the intended use of the medical sponge, a step of hydrating the biocompatible absorption material of the first layer may be included.

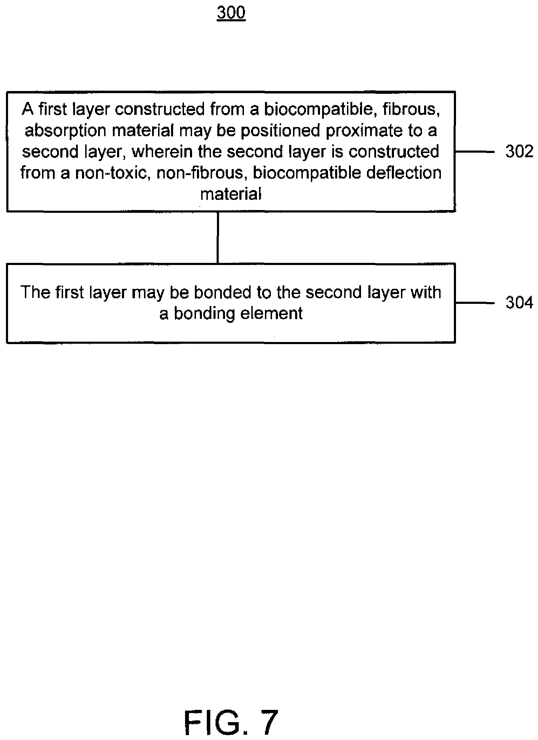

FIG. 7 is a flowchart 300 illustrating an exemplary method of constructing a medical sponge. It should be noted that any process descriptions or blocks in flow charts should be understood as representing modules, segments, portions of code, or steps that include one or more instructions for implementing specific logical functions in the process.

As is shown by block 302, a first layer 20 constructed from a biocompatible, fibrous, absorption material may be positioned proximate to a second layer 30, wherein the second layer 30 is constructed from a non-toxic, non-fibrous, biocompatible deflection material. The first layer 20 may be bonded to the second layer 30 with a bonding element 40 (Block 304).

It should be emphasized that the above-described embodiments of the present disclosure, particularly, any "preferred" embodiments, are merely possible examples of implementations, merely set forth for a clear understanding of the principles of the disclosure. Many variations and modifications may be made to the above-described embodiments of the disclosure without departing substantially from the spirit and principles of the disclosure. All such modifications and variations are intended to be included herein within the scope of this disclosure and the present disclosure and protected by the following claims.

## Claims

1. A medical sponge (10, 110) comprising:
a first layer (20, 120) constructed from a biocompatible absorption material, wherein the absorption material includes a woven or non-woven material;
a second layer (30, 130) positioned abutting the first layer (20, 120), the second layer (30, 130) constructed from a non-toxic, biocompatible deflection material that includes polytetrafluoroethylene (PTFE); and
a bonding element (40, 140) connected to the first layer (20, 120) and the second layer (30, 130), wherein the bonding element (40, 140) adheres the first layer (20, 120) and the second layer (30, 130) together and the bonding element (40, 140) is selected from the group consisting of: an ultrasonic welding joint, a plurality of stitches, or an adhesive-based bonding material, such as glues, or adhesive compounds.

2. The medical sponge (10, 110) of claim 1, further comprising a radiopaque marker (50) positioned between the first layer (20, 120) and the second layer (30, 130).

3. The medical sponge (10, 110) of claims 1 or 2, further comprising a positioning element (60) affixed to at least one of: the first layer (20, 120) and the second layer (30, 130).

4. The medical sponge (10, 110) of claim 3, wherein the positioning element (60) comprises at least one of: a flexible string and a substantially rigid tab.

5. The medical sponge (10, 110) of claims 3 or 4, further comprising an identification element (70) affixed to the positioning element (60).

6. The medical sponge (10, 110) of claim 1, further comprising an identification element (70) affixed to at least one of: the first layer (20, 120) and the second layer (30, 130).

7. The medical sponge (10, 110) of claims 1, 2, 3, 4, 5, or 6 wherein the deflection material of the second layer (30, 130) comprises a non-fibrous material.

## Patentansprüche

1. Medizinischer Schwamm (10, 110), umfassend:
eine erste Schicht (20, 120), die aus einem biokompatiblen Absorptionsmaterial konstruiert ist, wobei das Absorptionsmaterial einen Webstoff oder einen Vliesstoff beinhaltet;
eine zweite Schicht (30, 130), die angrenzend an die erste Schicht (20, 120) positioniert ist, wobei die zweite Schicht (30, 130) aus einem nichttoxischen, biokompatiblen Verformungsmaterial, das Polytetrafluorethylen (PTFE) beinhaltet, konstruiert ist; und
ein Bindungselement (40, 140), das mit der ersten Schicht (20, 120) und der zweiten Schicht (30, 130) verbunden ist, wobei das Bindungselement (40, 140) die erste Schicht (20, 120) und die zweite Schicht (30, 130) aneinander befestigt und das Bindungselement (40, 140) aus der Gruppe ausgewählt ist, die aus: einer Ultraschallschweißstelle, einer Vielzahl von Stichen oder einem Bindungsmaterial auf Klebemittelbasis, beispielsweise Klebstoffe, oder Klebemittelverbindungen besteht.

2. Medizinischer Schwamm (10, 110) nach Anspruch 1, ferner umfassend eine röntgendichte Markierung (50), die zwischen der ersten Schicht (20, 120) und der zweiten Schicht (30, 130) positioniert ist.

3. Medizinischer Schwamm (10, 110) nach Anspruch 1 oder 2, ferner umfassend ein Positionierungselement (60), das an mindestens einer der ersten Schicht (20, 120) und der zweiten Schicht (30, 130) angebracht ist.

4. Medizinischer Schwamm (10, 110) nach Anspruch 3, wobei das Positionierungselement (60) mindestens eines von einer flexiblen Schnur und einer im Wesentlichen starren Lasche umfasst.

5. Medizinischer Schwamm (10, 110) nach Anspruch 3 oder 4, ferner umfassend ein Identifizierungselement (70), das an dem Positionierungselement (60) angebracht ist.

6. Medizinischer Schwamm (10, 110) nach Anspruch 1, ferner umfassend ein Identifizierungselement (70), das an mindestens einer der ersten Schicht (20, 120) und der zweiten Schicht (30, 130) angebracht ist.

7. Medizinischer Schwamm (10, 110) nach Anspruch 1, 2, 3, 4, 5 oder 6, wobei das Verformungsmaterial der zweiten Schicht (30, 130) ein nichtfaseriges Material umfasst.

## Revendications

1. Éponge médicale (10, 110), comprenant :
une première couche (20, 120) fabriquée à partir d'un matériau d'absorption biocompatible, le matériau d'absorption comprenant un matériau tissé ou non-tissé ;
une deuxième couche (30, 130) agencée en butée contre la première couche (20, 120), la deuxième couche (30, 130) étant fabriquée à partir d'un matériau de déflexion biocompatible non-toxique qui comprend du polytétrafluoroéthylène (PTFE) ; et
un élément de liaison (40, 140) raccordé à la première couche (20, 120) et à la deuxième couche (30, 130), l'élément de liaison (40, 140) fixant la première couche (20, 120) et la deuxième couche (30, 130) l'une à l'autre, et l'élément de liaison (40, 140) étant choisi dans le groupe constitué : d'un joint de soudage par ultrasons, d'une pluralité de points de couture, ou d'un matériau de liaison à base d'adhésif tel que des colles, ou de composés adhésifs.

2. Éponge médicale (10, 110) selon la revendication 1, comprenant en outre un repère radio-opaque (50) agencé entre la première couche (20, 120) et la deuxième couche (30, 130).

3. Éponge médicale (10, 110) selon la revendication 1 ou 2, comprenant en outre un élément de positionnement (60) fixé : à la première couche (20, 120) et/ou à la deuxième couche (30, 130).

4. Éponge médicale (10, 110) selon la revendication 3, l'élément de positionnement (60) comprenant : un fil flexible et/ou une patte sensiblement rigide.

5. Éponge médicale (10, 110) selon la revendication 3 ou 4, comprenant en outre un élément d'identification (70) fixé à l'élément de positionnement (60).

6. Éponge médicale (10, 110) selon la revendication 1, comprenant en outre un élément d'identification (70) fixé : à la première couche (20, 120) et/ou à la deuxième couche (30, 130).

7. Éponge médicale (10, 110) selon les revendications 1, 2, 3, 4, 5 ou 6, le matériau de déflexion de la deuxième couche (30, 130) comprenant un matériau non-fibreux.
